# EUROPÄISCHE PATENTSCHRIFT

(11) **EP 0 785 980 B1**
(45) Veröffentlichungstag und Bekanntmachung des Hinweises auf die Patenterteilung: **10.03.1999**
(21) Anmeldenummer: 95933428.5
(22) Anmeldetag: 25.09.1995
(51) Int. Cl.: C11D 1/94, A61K 7/50

(54) **PUMPFÄHIGE WÄSSRIGE TENSIDKONZENTRATE**
PUMPABLE AQUEOUS TENSIDE CONCENTRATES
CONCENTRES DE TENSIOACTIFS AQUEUX POMPABLES

(30) Priorität: 04.10.1994 DE 4435387
(43) Veröffentlichungstag der Anmeldung: 30.07.1997
(73) Patentinhaber: Henkel Kommanditgesellschaft auf Aktien, 40191 Düsseldorf (DE)
(72) Erfinder: HENSEN, Hermann, D-42781 Haan (DE); LINDNER, Renate, D-40721 Hilden (DE); KAHRE, Jörg, D-40789 Monheim (DE); SEIPEL, Werner, D-40723 Hilden (DE)
(86) Internationale Anmeldenummer: EP9503795
(87) Internationale Veröffentlichungsnummer: WO9610622

(56) Entgegenhaltungen:
- EP-A- 0 127 580
- EP-A- 0 219 057
- EP-A- 0 250 181
- EP-A- 0 358 216
- EP-A- 0 409 005
- EP-A- 0 453 238
- EP-A- 0 572 776
- WO-A-91/04313
- DE-A- 4 139 935
- US-A- 4 668 422

## Beschreibung

### Gebiet der Erfindung

Die Erfindung betrifft pumpfähige, wäßrige Tensidkonzentrate mit einem Gehalt an Alkylglykosiden, Sulfosuccinaten und amphoteren Tensiden sowie deren Verwendung zur Herstellung von oberflächenaktiven Mitteln.

### Stand der Technik

Alkyloligoglykoside, insbesondere Alkyloligoglucoside, stellen nichtionische Tenside dar, die infolge ihrer ausgezeichneten Detergenseigenschaften und hohen ökotoxikologischen Verträglichkeit zunehmend an Bedeutung gewinnen. Herstellung und Verwendung dieser Stoffe sind gerade in letzter Zeit in einer Reihe von Übersichtsartikeln dargestellt worden, von denen stellvertretend die Veröffentlichungen von H.Hensen in **Skin Care Forum, 1, (Okt.1992),** D.Balzer und N.Ripke in **Seifen-Öle-Fette-Wachse 118, 894 (1992)** und B.Brancq in **Seifen- Öle-Fette-Wachse 118, 905 (1992)** genannt werden sollen.

In mancher Hinsicht ist der Einsatz von Alkyloligoglucosiden jedoch mit Problemen behaftet. So lassen sich beispielsweise keine pumpfähigen wäßrigen Konzentrate mit einem Feststoffgehalt oberhalb von 40 Gew.-% herstellen, ohne daß es im Zuge der Aufkonzentrierung zu einer partiellen Zersetzung der Zuckerkomponente kommt. Diese Eigenschaft haben die Glykoside übrigens mit den meisten anionischen Tensiden gemeinsam, die oberhalb eines Aktivsubstanzgehaltes von etwa 35 Gew.-% eine zähflüssige Gelphase bilden. Alkyloligoglucoside zeigen ferner die Tendenz, im Laufe einer Lagerung bei niedrigen Temperaturen zu kristallisieren, was ihre weitere Verwendung signifikant erschwert.

Aus dem Stand der Technik sind bereits eine Vielzahl von Tensidgemischen bekannt, die als eine Komponente Alkylglykoside enthalten, die oben genannten Probleme jedoch nicht zu lösen vermögen. So werden beispielsweise von G.Proserpio et al. in **Rivista Italiana 56**, **567 (1974)** Mischungen von kurzkettigen Alkylglykosiden mit Sulfosuccinaten zur Herstellung von Haarshampoos und Babyschaumbädern beschrieben. Abmischungen von Alkylglykosiden mit Sulfosuccinaten zum Eisatz als Sammler in der Flotation sind aus der **EP-A 0219057** (Henkel) bekannt. Gegenstand der **EP-A 0280143** (Henkel) sind manuelle Geschirrspülmittel mit einem Gehalt an Dioctylsulfosuccinaten. Milde Tensidgemische mit Sulfosuccinaten, vorzugsweise Monoalkylsulfosuccinaten, werden sehr breit in der **EP-A 0358216** (Kao) sowie der **WO-A-90/01441** (Henkel Corp.) beansprucht. Die Verwendung von Alkylglykosiden zur Viskositätsregulierung von Sulfosuccinatpasten wird in den Schriften **WO-A-91/04313** (Henkel Corp.) und **DE-A 4007757** (Henkel) vorgeschlagen. Aus den Schriften **DE-A 4139935** (Kao) und **EP-A 0572776** (Hüls) sind weiterhin flüssige Reinigungsmittel bekannt, die als Komponenten Alkylglykoside und Sulfosuccinate enthalten können. Schließlich werden in der **EP-A 0453238** (Unilever) milde Shampoos beansprucht, die anionische Tenside (z.B. Fettalkoholethersulfate und Sulfosuccinate), amphotere Tenside (z.B. Betaine) und nichtionische Tenside (z.B. Fettalkoholpolyglykolether und Alkylglykoside) enthalten, wobei der Feststoffgehalt der Mischungen jedoch deutlich unter 20 Gew.-% liegt. Zudem werden Kombinationen von Alkylglykosiden, Sulfosuccinaten und Amphotensiden nicht ausdrücklich genannt. Kombinationen von Alkylglykosiden mit amphoteren Tensiden vom Betaintyp sind beispielsweise Gegenstand der folgenden Druckschriften: **US-A-4,668,422** (Henkel Corp.), **EP-A 0250181** (Helene Curtis), **EP-A 0341071** (Unilever), **EP-A 0508507** (Berol Nobel) sowie **DE-A 4234487** und **DE-A 4311114** (Henkel). Aus der **EP-A 0409005** sind schließlich Zubereitungen mit Alkylglucosiden, Sulfosuccinaten und Betainen bekannt, die jedoch einen Feststoffgehalt unterhalb von 30 Gew.-% aufweisen.

Im Markt besteht ein Bedürfnis nach konzentrierten Tensidmischungen auf Basis von Alkyl- und/oder Alkenyloligoglucosiden, die bei einem Feststoffgehalt oberhalb von 30 Gew.-% und vorzugsweise von etwa 40 bis 45 Gew.-% fließ- und pumpfähig sind sowie eine signifikant verminderte Kristallisationsneigung, d.h. eine verbesserte Lagerstabilität aufweisen. Da derartige Tensidcompounds überwiegend im Kosmetiksektor Verwendung finden, ist die hautkosmetische bzw. dermatologische Verträglichkeit ebenfalls überaus wichtig. Die Tensidkonzentrate stellen für Hersteller und Anwender eine besonders vorteilhafte Handelsform dar, da sie hinsichtlich ihres Wassergehaltes minimiert worden sind und somit geringere Kosten bei Transport und Lagerung verursachen. Gleichwohl ist erwünscht, daß die Tensidkonzentrate bei Einsatz in den Endformulierungen, die naturgemäß stark verdünnt sind und einen Feststoffgehalt von 20 bis 30 Gew.-% aufweisen, eine ausreichend hohe Viskosität aufweisen bzw. sich problemlos unter Einsatz bekannter Zusatzstoffe verdicken lassen.

Die komplexe Aufgabe der Erfindung hat somit darin bestanden, pumpfähige wäßrige Tensidkonzentrate mit guter hautkosmetischer Verträglichkeit auf Basis von Alkyl- und/oder Alkenyloligoglykosiden zur Verfügung zu stellen, die sich durch eine hohe Lagerstabilität auszeichnen, eine Viskosität nach Brookfield von maximal 10.000 mPas sowie einen Feststoffgehalt von 30 bis 50 Gew.-% aufweisen und sich bei Einarbeitung in kosmetische Mittel mit einem Wassergehalt von mindestens 50 Gew.-% problemlos auf eine Viskosität von mindestens 2000 mPas verdicken lassen.

### Beschreibung der Erfindung

Gegenstand der Erfindung sind pumpfähige, wäßrige Tensidkonzentrate mit einem Feststoffgehalt von 30 bis 50 Gew.-%, enthaltend jeweils - bezogen auf den Feststoffgehalt - 25 bis 50 Gew.-%
(a) Alkyl- und/oder Alkenyloligoglykoside,
(b) Sulfosuccinate und
(c) amphotere bzw. zwitterionische Tenside,
mit der Maßgabe, daß sich die Mengen der drei Komponenten zu 100 Gew.-% ergänzen.

Überraschenderweise wurde gefunden, daß die erfindungsgemäßen Tensidkonzentrate eine ausgezeichnete hautkosmetische Verträglichkeit besitzen und über eine sehr gute Lagerstabilität auch bei niedrigen Temperaturen verfügen. Insbesondere die Ausbildung von Kristallen, wie sie von wäßrigen Alkylglucosidpasten bekannt ist, wird zuverlässig vermieden. Die Erfindung schließt ferner die Erkenntnis ein, daß die Tensidkonzentrate die erforderlich niedrige Viskosität von weniger als 10.000 und vorzugsweise 3.000 bis 7.500 mPas (bestimmt nach der Brookfield-Methode) aufweisen, sich jedoch in verdünnten wäßrigen Formulierungen problemlos auf eine Viskosität von mindestens 2.000 mPas verdicken lassen.

### Alkyl- und/oder Alkenyloligoglykoside

Alkyl- und/oder Alkenyloligoglykoside stellen bekannte Stoffe dar und folgen der Formel **(I)**,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Alkyl- und/oder Alkenyloligoglykoside können nach den einschlägigen Verfahren der präparativen organischen Chemie erhalten werden; stellvertretend für das umfangreiche Schrifttum sei hier auf die Schriften **EP-A1 0301298** und **WO-A-90/03977** verwiesen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/ oder Alkenyloligoglykoside sind somit Alkyl- und/oder Alkenyloligo**glucoside**. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich femer auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Sulfosuccinate

Sulfosuccinate, die auch als Sulfobemsteinsäureester bezeichnet werden, stellen bekannte anionische Tenside dar, die nach den einschlägigen Methoden der präparativen organischen Chemie erhalten werden können. Sie folgen der Formel **(II)**, 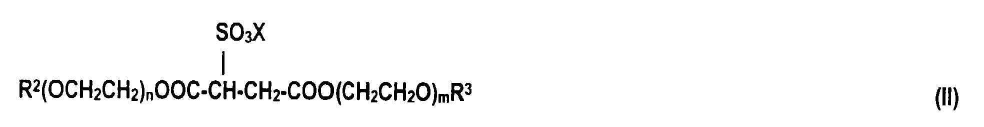 in der R² für einen Alkyl- und/oder Alkenylrest mit 6 bis 22 Kohlenstoffatomen, R³ für R¹ oder X, m und n unabhängig voneinander für 0 oder Zahlen von 1 bis 10 und X für ein Alkali- oder Erdalkalimetall, Ammonium, Alkylammonium, Alkanolammonium oder Glucammonium steht. Zu ihrer Herstellung geht man üblicherweise von Maleinsäure, vorzugsweise aber Maleinsäureanhydrid aus, die im ersten Schritt mit gegebenenfalls ethoxylierten primären Alkoholen verestert werden. An dieser Stelle kann durch Varia-tion von Alkoholmenge und Temperatur das Mono-/Diester-Verhältnis eingestellt werden. Im zweiten Schritt erfolgt die Anlagerung von Bisulfit, die üblicherweise im Lösungsmittel Methanol durchgeführt wird. Neuere Übersichten zu Herstellung und Verwendung von Sulfosuccinaten sind beispielsweise von T.Schoenberg in **Cosm.Toil. 104, 105 (1989)**, J.A.Milne in **R.Soc.Chem. (Ind.Appl.Surf.II) 77**, **77 (1990)** sowie W.Hreczuch et al. in **J.Am.Oil.Chem.Soc. 70**, **707 (1993)** erschienen. Typische Beispiele sind Sulfobemsteinsäuremono- und/oder -diester in Form ihrer Natriumsalze, die sich von Fettalkoholen mit 8 bis 18, vorzugsweise 8 bis 10 bzw. 12 bis 14 Kohlenstoffatomen ableiten; die Fettalkohole können dabei mit durchschnittlich 1 bis 10 und vorzugsweise 1 bis 5 Mol Ethylenoxid verethert sein und dabei sowohl eine konventionelle als auch vorzugsweise eine eingeengte Homologenverteilung aufweisen. Exemplarisch genannt seien Di-n-octylsulfosuccinat und Monolauryl-3EO-sulfosuccinat in Form ihrer Natriumsalze.

### Amphotere bzw. zwitterionische Tenside

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. In einer besonders bevorzugten Ausführungsform der Erfindung werden als amphotere Tenside Kondensationsprodukte von Fettsäureamidoaminen mit Halogencarbonsäuresalzen eingesetzt, die der Formel **(III)** folgen, 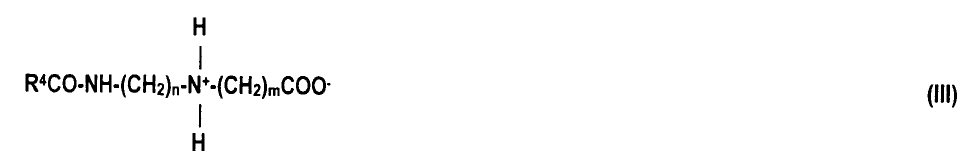 in der R⁴CO für einen gesättigten und/oder ungesättigten aliphatischen Acylrest mit 6 bis 22, vorzugsweise 12 bis 18 Kohlenstoffatomen und n und m unabhängig voneinander für Zahlen von 1 bis 3 stehen. Typische Beispiele sind Kondensationsprodukte von Natriumchloracetat mit Amidoamiden der Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind Kondensationsprodukte von C_{12/14}- bzw. C_{8/18}-Kokosfettsäureamidoethylenamin bzw. -propylenamin mit Natriumchloracetat.

Die erfindungsgemäßen Tensidkonzentrate enthalten die Komponenten (a), (b) und (c) jeweils in Mengen von 25 bis 50, vorzugsweise 30 bis 40 Gew.-%, mit der Maßgabe, daß sich die Angaben jeweils zu 100 Gew.-% ergänzen. Ferner weisen die Gemische in der Regel einen Wassergehalt von mindestens 50 und höchstens 70, vorzugsweise 55 bis 60 Gew.-% auf.

### Gewerbliche Anwendbarkeit

Die erfindungsgemäßen wäßrigen Tensidkonzentrate weisen einen Feststoffgehalt im Bereich von 30 bis 50 Gew.-% auf. Sie sind lagerstabil, neigen nicht zur Kristallisation, besitzen eine Viskosität von weniger als 10.000 mPas und sind dementsprechend pumpfähig. In verdünnten wäßrigen Formulierungen zeigen sie eine gute Wiederverdickbarkeit und eine ausgezeichnete hautkosmetische Verträglichkeit. Sofem die Tensidkonzentrate bereits ein Verdickungsmittel enthalten, kann in vielen Fällen auf den weiteren Zusatz von Verdickungsmitteln, vorzugsweise Fettalkoholpolyglycolethem mit eingeengter Homologenverteilung, zu den kosmetischen Mitteln sogar verzichtet werden, da sich die gewünschte Viskosität automatisch einstellt. Ein weiterer Gegenstand der Erfindung betrifft daher die Verwendung der erfindungsgemäßen Tensidkonzentrate zur Herstellung von oberflächenaktiven Mitteln, wie z.B. Handgeschirrspülmitteln sowie insbesondere Mitteln zur Haut- und Haarpflege.

### Haut- und Haarpflegemittel

**Haut- und Haarpflegemittel** auf Basis der erfindungsgemäßen Tensidkonzentrate können in untergeordneten Mengen weitere, mit den anderen Inhaltsstoffen kompatible **Tenside** enthalten. Typische Beispiele sind Fettalkoholpolyglycolethersulfate, Monoglyceridsulfate, Ethercarbonsäuren, Fettsäureisethionate, Fettsäuridesarcosinate, Fettsäuretauride, und/oder Proteinhydrolysate bzw. deren Kondensate mit Fettsäuren auf tierischer oder vorzugsweise pflanzlicher Basis.

**Hautpflegemittel,** wie Cremes, Lotionen und dergleichen, weisen in der Regel - neben den bereits genannten Tensiden - einen Gehalt an Ölkörpem, Emulgatoren, Fetten und Wachsen, Stabilisatoren sowie ebenfalls Überfettungsmitteln, Verdikkungsmitteln, biogenen Wirkstoffen, Filmbildnem, Konservierungsmitteln, Farb- und Duftstoffen auf. **Haarpflegemittel**, wie beispielsweise Haarshampoos, Haarlotionen, Schaumbäder und dergleichen, können als weitere Hilfs- und Zusatzstoffe - neben den bereits genannten Tensiden - Emulgatoren, Überfettungsmittel, Verdickungsmittel, biogene Wirkstoffe, Filmbildner, Konservierungsmittel, Farb- und Duftstoffe enthalten.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₁₆-C₁₈-Fettalkoholen, Ester von linearen C₁₀-C₁₈-Fettsäuren mit verzweigten Alkoholen, insbesondere 2-Ethylhexanol, Ester von linearen und/oder verzweigten Fettsäuren mit zweiwertigen Alkoholen und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate und/oder Dialkylether in Betracht.

Als **Emulgatoren** kommen sowohl bekannte W/O- als auch O/W- Emulgatoren, wie beispielsweise gehärtetes und ethoxyliertes Ricinusöl, Polyglycerinfettsäureester oder Polyglycerinpolyricinoleate in Frage. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Stabilisatoren** können Metallsalze von Fettsäuren wie z.B. Magnesium-, Aluminium- und/oder Zinkstearat eingesetzt werden. Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid. Unter **biogenen Wirkstoffen** sind beispielsweise Pflanzenextrakte und Vitaminkomplexe zu verstehen. Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% und der nicht wäßrige Anteil ("Aktivsubstanzgehalt") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

### Beispiele

Die Viskosität der folgenden Gemische wurde nach der Brookfield-Methode (20°C, Spindel 4, 10 UpM) bestimmt. Die Stabilität der Gemische wurde nach Lagerung über 4 Wochen bei 5°C visuell beurteilt. Dabei bedeuten:
- (+++) =: klare Lösung, keine Kristallbildung
- (++) =: vereinzelte Kristallbildung
- (+) =: deutliche Kristallbildung
- (-) =: Teile der Lösung durchkristallisiert
- (*) =: trübes Produkt, instabil, Phasentrennung

Anschließend wurden die Mischungen in die Rezeptur einer verdünnten kosmetischen Hautlotion der folgenden Zusammensetzung eingesetzt (Wasser ad 100 Gew.%): 10 Gew.-% Tensidkonzentrat (berechnet wasserfrei), 5 Gew.-% Kokoamidopropylbetain (Dehyton® K), 2,5 Gew.-% Potassium Kokoyl hydrolisiertes Kollagen (Lamepon® S), 1,5 Gew.-% PEG-3 Distearat und Natrium Laurethsulfat (Euperlan® PK 900), 1,0 Gew.-% PEG-7 Glycerylkokoat (Cetiol® HE), 2,5 Gew.-% Glyceryllaurat und Potassium Kokoyl hydrolisiertes Kollagen (Lamesoft® LMG), 1,0 Gew.-% Farbstoff und 0,1 Gew.-% Konservierungsmittel. Die Zusammensetzungen sowie die Daten zur Viskosität und zur Stabilität sind in Tabelle 1 zusammengefaßt. Die Angaben zu den Zusammensetzungen der Gemische (in Gew.-%) verstehen sich bezogen auf den Feststoffgehalt der Komponenten. Alle Gemische wurden mit einem Wassergehalt von 55 Gew.-% (pH-Wert 5,3) eingesetzt. Mischung 1 ist erfindungsgemäß, die Mischungen V1 bis V5 dienen zum Vergleich.

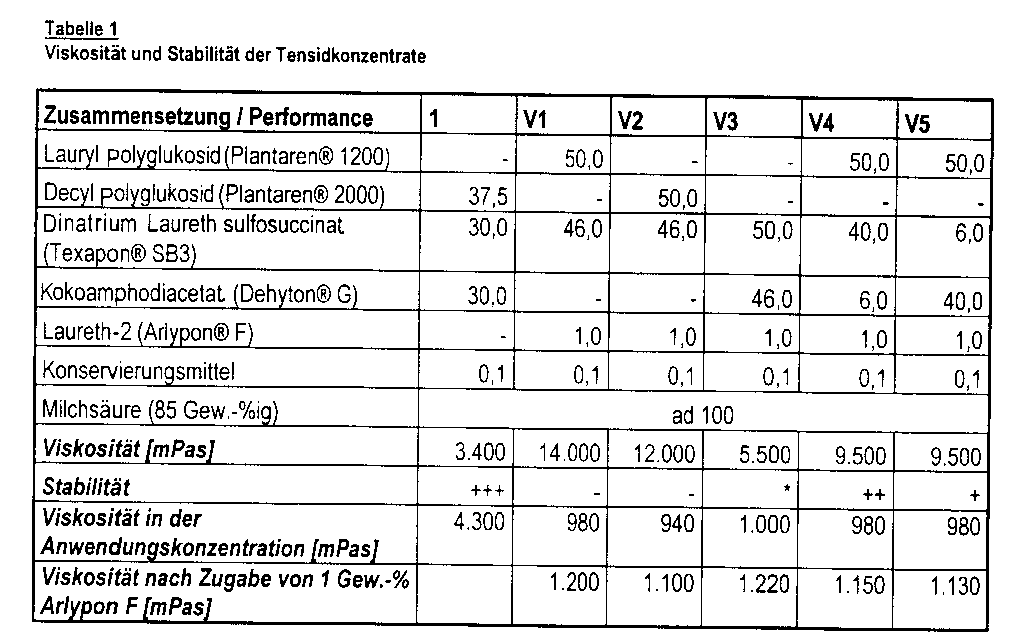

Die Beispiele und Vergleichsversuche lassen sich wie folgt zusammenfassen:
- Konzentrierte Mischungen von Alkyloligoglucosiden und Sulfosuccinaten ergeben zähflüssige bis schnittfeste Pasten. Die auf der Grundlage dieser Konzentrate hergestellten verdünnten kosmetischen Formulierungen weisen jedoch eine sehr niedrige Viskosität auf, die sich mit konventionellen Verdickungsmitteln nicht auf den gewünschten Wert anheben läßt.
- Konzentrierte Mischungen von Sulfosuccinaten und Amphotensiden ergeben dünnflüssige, instabile Lösungen. Bei Einarbeitung in die verdünnten kosmetischen Formulierungen resultieren ebenfalls sehr niedrigviskose Produkte, die sich nicht in gewünschter Weise verdicken lassen.
- Nur die erfindungsgemäßen Tensidkonzentrate weisen eine ausreichend niedrige Viskosität bei optimalem Lagerverhalten auf und lassen sich in der verdünnten Anwendung problemlos auf die gewünschte Viskosität einstellen.

## Patentansprüche

1. Pumpfähige, wäßrige Tensidkonzentrate mit einem Feststoffgehalt von 30 bis 50 Gew.-%, enthaltend jeweils - bezogen auf den Feststoffgehalt - 25 bis 50 Gew.-%
(a) Alkyl- und/oder Alkenyloligoglykoside,
(b) Sulfosuccinate und
(c) amphotere bzw. zwitterionische Tenside,
mit der Maßgabe, daß sich die Mengen der drei Komponenten zu 100 Gew.-% ergänzen.

2. Tensidkonzentrate nach Anspruch 1, **dadurch gekennzeichnet**, daß sie Alkyl- und/oder Alkenyloligoglykoside der Formel **(I)** enthalten,
**R**^{**1**}**O-[G]**_{**p**} **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Tensidkonzentrate nach Anspruch 1, **dadurch gekennzeichnet,** daß sie Sulfosuccinate der Formel **(II)** enthalten, 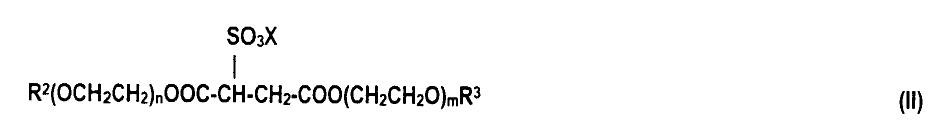 in der R² für einen Alkyl- und/oder Alkenylrest mit 6 bis 22 Kohlenstoffatomen, R³ für R¹ oder X, m und n unabhängig voneinander für 0 oder Zahlen von 1 bis 10 und X für ein Alkali- oder Erdalkalimetall, Ammonium, Alkylammonium, Alkanolammonium oder Glucammonium steht.

4. Tensidkonzentrate nach Anspruch 1, **dadurch gekennzeichnet**, daß sie amphotere bzw. zwitterionische Tenside enthalten, die ausgewählt sind aus der Gruppe, die von Alkylbetainen, Alkylamidobetainen, Aminopropionaten, Aminoglycinaten, Imidazoliniumbetainen und Sulfobetainen gebildet wird.

5. Tensidkonzentrate nach den Ansprüchen 1 bis 4, **dadurch gekennzeichnet,** daß sie die Komponenten (a), (b) und (c) jeweils - bezogen auf den Feststoffgehalt - in Mengen von 30 bis 40 Gew.-% enthalten, mit der Maßgabe, daß sich die Mengen der drei Komponenten zu 100 Gew.-% ergänzen.

6. Tensidkonzentrate nach den Ansprüchen 1 bis 5, **dadurch gekennzeichnet**, daß sie einen Wassergehalt von 50 bis 70 Gew.-% aufweisen.

7. Verwendung der Tensidkonzentrate nach den Ansprüchen 1 bis 6 zu Herstellung von oberflächenaktiven Mitteln.

## Claims

1. Pumpable water-containing surfactant concentrates with a solids content of 30 to 50% by weight containing - based on the solids content - 25 to 50% by weight of each of the following components:
(a) alkyl and/or alkenyl oligoglycosides,
(b) sulfosuccinates and
(c) amphoteric or zwitterionic surfactants,
with the proviso that the quantities of the three components add up to 100% by weight.

2. Surfactant concentrates as claimed in claim 1, characterized in that they contain alkyl and/or alkenyl oligoglycosides corresponding to formula (I):
R¹O-[G]ₚ (I)
in which R¹ is an alkyl and/or alkenyl radical containing 4 to 22 carbon atoms, G is a sugar unit containing 5 or 6 carbon atoms and p is a number of 1 to 10.

3. Surfactant concentrates as claimed in claim 1, characterized in that they contain sulfosuccinates corresponding to formula (II): 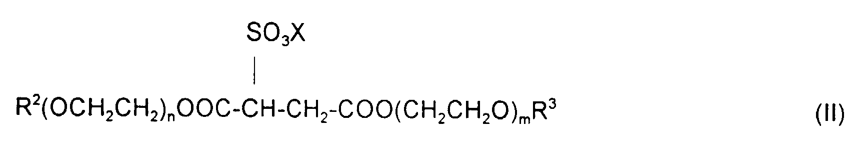 in which R² is an alkyl and/or alkenyl radical containing 6 to 22 carbon atoms, R³ has the same meaning as R¹ or X, m and n independently of one another are 0 or numbers of 1 to 10 and X is an alkali metal or alkaline earth metal, ammonium, alkylammonium, alkanolammonium or glucammonium.

4. Surfactant concentrates as claimed in claim 1, characterized in that they contain amphoteric or zwitterionic surfactants selected from the group formed by alkyl betaines, alkyl amidobetaines, aminopropionates, aminoglycinates, imidazolinium betaines and sulfobetaines.

5. Surfactant concentrates as claimed in claims 1 to 4, characterized in that they contain each of components (a), (b) and (c) in quantities - based on the solids content - of 30 to 40% by weight, with the proviso that the quantities of the three components add up to 100% by weight.

6. Surfactant concentrates as claimed in claims 1 to 5, characterized in that they have a water content of 50 to 70% by weight.

7. The use of the surfactant concentrates claimed in claims 1 to 6 for the production of surface-active formulations.ed in dilute cosmetic formulations.

## Revendications

1. Concentrés tensioactifs aqueux pompables ayant une teneur en solides de 30 à 50 % en poids, contenant toujours - par rapport à la teneur en solides - de 25 à 50 % en poids
(a) D'alkyl- et/ou alcényloligoglycosides,
(b) De sulfosuccinates et
(c) D'agents tensioactifs amphotères ou selon les cas hermaphrodites,
sous réserve que les quantités des trois composants se complètent à 100 %.

2. Concentrés d'agents tensioactifs selon la revendication 1,
caractérisés en ce qu'ils contiennent des alkyl- et/ou alcényloligoglycosides de formule **(I)**,
**R**^{**1**}**O-[G]**_{**p**} **(I)**
dans laquelle R¹ représente un radical alkyl et/ou alcényle ayant de 4 à 22 atomes de carbone, G représente un radical saccharique ayant 5 ou 6 atomes de carbone, et p représente des nombres compris entre 1 et 10.

3. Concentrés d'agents tensioactifs selon la revendication 1,
caractérisés en ce qu'
ils contiennent des sulfosuccinates de formule **(II)** 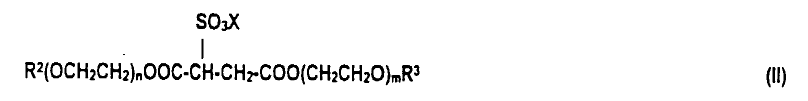 dans laquelle R² représente un radical alkyle et/ou alcényle ayant de 6 à 22 atomes de carbone, R³ représente R¹ ou X, m et n valent indépendamment l'un de l'autre 0 ou des nombres compris entre 1 et 10 et X représente un métal alcalin ou alcalino-terreux, l'ammonium, un alkylammonium, un alcanolammonium ou le glucammonium.

4. Concentrés d'agents tensioactifs selon la revendication 1,
caractérisés en ce qu'
ils contiennent des agents tensioactifs amphotères ou selon les cas hermaphrodites qui sont choisis dans le groupe formé par les alkylbétaïnes, les alkylamidobétaïnes, les aminopropionates, les aminoglycinates, les imidazoliniumbétaïnes et les sulfobétaïnes.

5. Concentrés d'agents tensioactifs selon les revendications 1 à 4,
caractérisés en ce qu'
ils contiennent les composants (a), (b) et (c), toujours - par rapport à la teneur en solides - à des quantités de 30 à 40 % en poids, sous réserve que les quantités des trois composants se complètent à 100 % en poids.

6. Concentrés d'agents tensioactifs selon les revendications 1 à 5,
caractérisés en ce qu'
ils ont une teneur en eau de 50 à 70 % en poids.

7. Utilisation des concentrés tensioactifs selon les revendications 1 à 6 pour la production d'agents tensioactifs.
